# EUROPEAN PATENT SPECIFICATION

(11) **EP 1 776 019 B1**
(45) Date of publication and mention of the grant of the patent: **23.03.2011**
(21) Application number: 04770757.5
(22) Date of filing: 12.08.2004
(51) Int. Cl.: A61K 36/06

(54) **NATURAL COMPOSITION**
NATÜRLICHE ZUSAMMENSETZUNG
COMPOSITION NATURELLE

(43) Date of publication of application: 25.04.2007
(73) Proprietor: RE.NA.CO. S.A.S. DI RAVANELLO F. & C., 31035 Crocetta del Montello (TV) (IT)
(72) Inventor: BIANCHI, Antonio, I-24065 Lovere, BERGAMO (IT)
(74) Representative: Long, Giorgio
(86) International application number: PCT/IT2004/000458
(87) International publication number: WO 2006/016383

(56) References cited:
- US-A- 4 563 362
- ANONYMOUS: "Cholest-Away" INTERNET ARTICLE, [Online] 18 February 2003 (2003-02-18), page 1, XP002326729 Retrieved from the Internet: URL:http://web.archive.org/web/20030218103 543/http://fortius.ca/cholestaway.htm> [retrieved on 2005-04-29] -& ANONYMOUS: "Page from Internet Archive Wayback Machine" INTERNET ARTICLE, [Online] page 1, XP002326731 Retrieved from the Internet: URL:http://web.archive.org/web/*/http://ww w.fortius.ca/cholestaway.htm> [retrieved on 2005-04-29]
- DAVID SCHARDT: "Cholesterol-Lowering Supplements" INTERNET ARTICLE, [Online] November 1997 (1997-11), page 1, XP002326730 Retrieved from the Internet: URL:http://www.cspinet.org/nah/novnah.htm> [retrieved on 2005-04-28]
- ANONYMOUS: "Monascus" INTERNET ARTICLE, [Online] pages 1-10, XP002326680 Retrieved from the Internet: URL:http://www.allok.com/deutsch/monascus_ monograph.html> [retrieved on 2005-04-29]

## Description

This invention relates to the use of a composition comprising Monascus fungus extract and grape extract as a drug for the control of the plasmatic levels of lipids and sugars, blood pressure and the inflammation of the cariovascular system.

The increase in triglycerides and cholesterol in the blood and particularly LDL cholesterol (low-density lipoproteins) is universally considered as an important risk factor for the development of cardiovascular diseases, which are the main cause of death in the modern world (Lipid Research Clinics Program 1984). In fact, it seems that the excess of lipids in the blood may favour their deposition on the blood vessel walls, thus activating the process leading to atherosclerosis. As a consequence, entire classes of drugs have been developed to reduce the lipid values in the blood: the statin for the cholesterol values and the fibrates for those of the triglycerides. The first statin, the lovastatin, has been isolated starting from a Chinese mushroom conventionally employed for the fermentation of the red rice wine, the Monascus purpureus.

In 1979 in Japan, prof. Akira Endo isolated the monacolin K from cultures of Monascus purpureus, and proved that this was able to inhibit the 3-hydroxy-3-methylglutarylcoenzymeA reductase (HMG-CoA reductase), an enzyme which is the 21st step in the synthesis of cholesterol (Endo A., Journ. of Antibiotics, 1980, 33(3), 334-336).

Without this enzyme, the whole synthesis sequence of cholesterol is stopped. As a consequence, adjusting the cholesterol values in the blood is an elective target. Subsequently, Endo established the identity between the monacolin K and mevinolin (or lovastatin), the precursor of those class of drugs, the statins, which are still nowadays considered as the elective treatment for hyperlipidemia and one of the most important categories of drugs available. For several years, the Monascus purpureus has become one of the sources of statins and an in vitro culture technique has been developed to the purpose, which is still used nowadays (Chang Y.N., Lin Y.C., Lee C.C., Tzeng Y.M.; Folia Microbiol., 2002, 47(6), 677-684).

Nowadays, statins are directly produced either by chemical synthesis or by other mushrooms, the Monascus is used as a hypolipidemic natural product at a monacolin concentration ranging between 0,03% and 2%.

One of the factors contributing to make the excess of lipids more dangerous is undoubtedly the possibility that they may undergo a peroxidation with a consequent accumulation of peroxides and free radicals. The oxidation of fats in the blood has been related to the occurrence of an inflammatory state in our arteries (Epstein F.H.; New Engl. Journ. of Medicine, 1997, 337(6), 408-416) and the beginning of the atherosclerotic process.

In atherosclerosis, the inflammatory mechanisms are becoming more and more important (Ross R.; New Engl. Journ. of Medicine, 1999, 340, 115-126). The excess and oxidation of the blood lipids is likely to favour the adhesion of monocytes and lymphocytes T to the endothelium (Toussoulis D., Davies G., Ambrose J., Tentolouris, Stefanadis C., Toutouzas P.; International Journal of cardiology, 2002, 86,239-247). Once this mechanism has started, the macrophages derived from monocytes and the lymphocyte cells release in turn a wide range of chemotactic and chemoactive substances. The endothelium becomes reactive, and this is exhibited through the expression of a number of factors such as interleukines, complement components, tumour necrosis factor, all of which being substances that contribute to amplify the inflammatory state by a cascade mechanism.

The importance of defining and monitoring the inflammatory state of our arteries is the reason behind which certain phlogosis parameters have become more and more important for the diagnosis of atherosclerosis and above all the individuation of the most effective therapies (Libby P., Rider P. M.; Am. Journ. Of Medicine, 2004, 116(6A), 9S-16S). Among these, the C-reactive protein is undoubtedly the most reliable one. The lipoprotein A (Lp(a)), due to its similarity with the plasminogen, is also likely to act an important role in establishing a connection between the inflammatory and thrombotic processes (Toussoulis D., Davies G., Ambrose J., Tentolouris, Stefanadis C., Toutouzas P.; International Journal of cardiology, 2002, 86,239-247). A recent study has proved that the interaction between Lp(a) and macrophages implies more action sites synergizing to attain a much more active interaction than that observed between cholesterol LDL and plasminogen (Poon M., Zhang X., Dunsy K. G., Tauban M. B. and Harpel P. C.; Circulation, 1997, 96, 2514-2519).

Precisely the route of thrombin activation may costitute an important mechanism in the determination of the spontaneous coronary occlusion in the atherosclerotic subjects (Haider A. W., Andreotti F., Thompson G. R., Iuft C., Masseri A., Davies G. I.; Circulation, 1997, 94, 2072-2076). The available studies disclose that, though exhibiting a good activity on the C-reactive protein, the statins have a poor activity on the lipoprotein system (a) (Saltissi D., Morgan C., Rigby R. J., Westhuyzen J.; Am J. Kidney Dis., 2002, 39, 293-290), when not leading to a real increase in this risk factor (Galetta F., Sampietro T., Basta G., Giannasi G. and Bionda A.; Minerva Medica, 1995, 86(7-8), 299-303).

Therefore, despite their powerful anti-hypercholesterolemizing activity, the statins also have negative effects, the improvement of which may constitute an advance towards a more efficient control of the different risk factors related to the pathogenesis of atherosclerosis. The action of the endogenous antioxidant substances may play not a minor role. Statins and fibrates, i.e. the hypolipidemizing drugs employed nowadays, seem to be the most limited in this aspect: statins lead to the depletion of important endogenous antioxidant substances such as the ubiquinone (coenzyme Q10) (Mortensen S. A., Leth A., AGNER, Rhode M.; Molec. Aspects Med., 1997, 18, s137-s1). The same effect is provided by the fibrates (Aberg F., Appelkvist A., Brojiersen A., Eriksson M., Angelin B., HJemdahl P., Dallner G.; (1998) J. Clinical Invenstigation, 28, 235-242): actually, both substances rather seem to contribute to a depletion of the antioxidant defences of our organism. Particularly, the Coenzyme Q is a key coenzyme in the mitochondrion respiratory chain. A low level of coenzyme Q is the symptom and cause, at the same time, of a mitochondrion dysfunction which generally translates into a cell energetic disequilibrium which is exhibited by an accumulation of piruvate/lactate (De Pineux P. , Chariot P., Ammi-Said M., Louarn F., Lejonc J. L., Astier A., Jacotot B., Gherardi R.; Br.Journ. Clin. Pharmacol., 1997, 42, 333-337). Probably, a dysfunction at the mitochondrion level is the responsible for the toxicity of the statins on the muscular cells (rhabdomyolysis).

Due to the presence of polyphenols, the grape extracts, essentially consisting of procyanidins and proanthocyanidins, can carry out a powerful inhibitory action on the lipid oxidation (Manijeh-Shafiee et al.; Free Radical Research, 1997, 37(5), 573-584), and carry out an alternative and complementary action to that of Coenzyme Q.

"Cholest-Away" INTERNET ARTICLE, [Online] 18 February 2003 (2003-02-18) , page 1, XP002326729 Retrieved from the Internet: URL:http://web.archive.org/web/20030218103543/http://fortius.ca/cholestaway.htm [retrieved on 2005-04-29] discloses a natural supplement comprising niacin, plant sterol concentrate, guggul gum extract, rosehips extract, red yeast extract (**Monascus Purpureus**), royal jelly powder and **grape seed extract.** This supplement is designed to aid in the fight against elevated cholesterol levels and the risk of cardiovascular disease.

The problem addressed by the present invention is to use a composition that does not exhibit the side effects of the statins, that improves the anti-inflammatory activity thereof, that performs an equivalent hypocholesterolemizing action and that improves the reduction of triglycerides, without causing depletion of important endogenous antioxidant substances such as the coenzyme Q10. The present invention is addressed to such an object as described and claimed in the annexed claims herein below.

The present invention relates to the use of a composition comprising Monascus Fungus extract and grape extract for preparing a medicament for the treatment and prevention of the accumulation of high levels of sugars in the blood.

The present invention relates also to the use of a composition comprising Monascus Fungus extract and grape extract for preparing a medicament for the treatment and prevention of the cardiovascular system inflammation.

The present invention relates also to the use of a composition comprising Monascus Fungus extract and grape extract for preparing a medicament for the treatment and prevention of hypertension.

Said medicament is also used for the treatment and prevention of diseases associated to the above cases, such as: hyperlipidemia, cardiac diseases, hypertension, atherosclerosis and metabolic disequilibrium.

Preferably, said composition comprises Monascus Fungus extract and grape seed extract.

Said Monascus Fungus extract comprises monacolin and further moieties comprising, inter alia: polyunsaturated fatty acids, phytosterols (es: β-sitosterol, capesterol, stigmasterol), isoflavons and glucosides thereof, saponins, various red pigments etc. The monacolins are a family of terpenic substances, belonging to the class of statins, classified based on their sub-unities into: monacolin K, J, L, X and M. Monacolin K is structurally identical to lovastatin.

Said Monascus Fungus extract comprises the total monacolin and particularly the monacolin K (or lovastatin) in amounts from 0,01% to 10% by weight, preferably from 0,2% to 5% by weight, more preferably from 0,3% to 2% by weight.

Said Monascus Fungus extract can be obtained from any species in the Monascus Fungus genus.

Preferably, the species that can be suitably used are: Monascus albidus Sato; Monascus pilosus Sato; Monascus pubigereus Sato; Monascus ruber van Tieghen; Monascus paxii Liengelsheim; Monascus fulginosus Sato; Monascus purpueus Went ecc. Most preferred species are: Monascus purpureus Went, Monascus ruber van Tieghen, Monascus fulginosus Sato, Monascus albidus Sato; still more preferred are: Monascus purpureus Went, Monascus ruber van Tieghen; the most preferred species is Monascus purpureus Went.

All species and sub-species of Monascus Fungus are commercially available.

The Monascus Fungus is grown and treated for extraction by any method known in the art. Some growing methods for Monascus Fungus are listed below, by way of example:
Kris T.; Production of Monascus Pigments Using Soy-soaking Water as Substrate; in Woll W. and Corcle SS (1978); Soybean as a Food Spurce; Butterworths, London.oll;
Wang R. e Webb C. (1997): Development of a generic fermentation feed stock from whole wheat flour. In C ampbell O., Webb C. and McKee S. L. (eds). Cereals: Novel Uses and Processes. Plnum Press, New York pp.205-218;
Su Y. C., Wang J. J., Lin T. T. and Pan T. M.; J. Ind. Microbiol. Biotechnol., 2003, 30, 41-46.

An example of how preparing the Monascus Fungus extract can be found in the following publication: Chang Y. N., Lin Y. C., Lee C. C., Tzeng Y. M.; Folia Microbiol., 2002, 47(6), 677-684.

Said grape extract comprises 1% to 99% by weight of polyphenols, preferably 50% to 99% by weight, more preferably 70% to 99%. Said polyphenols comprise 1% to 99% by weight of proanthocyanidins, preferably 70% to 95% by weight, more preferably 80% to 95% by weight and 1% to 99% by weight of anthocyanosides, preferably 5% to 30% by weight, more preferably 5% to 20% by weight.

Said grape extract can be obtained from any plant belonging to the family of Vitaceae, preferably from Vitis vinifera and preferably from the seeds of this vine, by using any known technique or it is commercially available.

The extraction of grape seeds can be, for example, carried out by the supercritical Co₂ sequential extraction method such as described by Ashraf-Khrassani M. and Taylor L. T., J. Agric. Food Chem., 2004, 52, 2440-2444.

Preferably, the grape extract used in the present invention is commercially available.

Said composition comprises Monascus Fungus extract in amounts from 7 % to 99 % by weight, preferably 21% to 95% by weight, more preferably 37% to 91% by weight and grape extract from 1% to 93% by weight, preferably 5% to 79% by weight, more preferably 9% to 63% by weight. The component amounts to be employed in said composition depend on the amount of active ingredients contained in the extracts (monacolin K and polyphenols). Obviously, the greater the contents, for example, of monacolin, the lower the amount of Monascus Fungus extract employed. For example, a Monascus Fungus extract, containing about 0,8% of monacolin K, and a grape extract, containing about 95% of polyphenols, may be employed in amounts of about 80% and 20% by weight, respectively.

The composition used may comprise one or more grape extracts and one or more Monascus Fungus extracts having different compositions. In this case, the amounts to be used can be easily adjusted by those of ordinary skill in the art.

### USE AND ADVANTAGES

The association of Monascus fungus extract and grape extract has surprising effects which are probably due to a synergic interaction between the various components of both extracts. These effects, which have been obtained by clinical studies on humans, are discussed in detail herein below:
- hypolipidemizing effect particularly on pre-and postprandial triglycerides. The reduction in cholesterol obtained by this association can be compared to that occurring following the administration of the statin-based drugs currently on the market. However, it should be evidenced that the preferred amount of statins administered with the inventive composition is far lower than the normally used doses (for example, the marketed statins-based preparations contain about 10- 80 mg of statins per daily dose; a typical dose of statins of the present invention is about 2.16 mg of monacolin which supplies the same effects as a dose of 20-40 mg of lovastatin). Accordingly, the effect obtained by using the composition is surprising and can be attributed to a synergic action between the combination of substances present in both extracts, the mechanism of which has not yet been clarified. Moreover, the effect of reducing the triglycerides is surprising since statins are known to have a partial and limited action on this parameter, when they are administered alone;
- anti-inflammatory effect evidenced by the reduction of the arterial inflammation parameters (particularly Lipoprotein (a) and TNF-alfa, but also C-reactive protein), greater than that obtained by administering the corresponding dose of statins or grape extract; also in this case, the effect is explicable only by a synergic action between the combination of substances composing both extracts;
- non-depletion effect of the organism's antioxidant defences (coenzyme Q), which is surprising in that it is contrary to the normal behaviour of statins, when administered alone;
- hypotensive effect in hypertensive subjects; this effect has never been observed when the statins and the grape extract are administered alone to this dosages;
- normalizing effect on the glycemia and glycemic metabolism; this effect has never been observed when the statins or the grape extract are administered alone to this dosages.

Accordingly, the association between grape extract and Monascus Fungus extract has a surprising synergic effect and is advantageous in that it has a low concentration of statins and, therefore, does not exhibit those negative effects linked to high doses of statins.

### FORMULATION AND DOSAGE

The composition used can be administered by oral, transdermal, or buccal route. In any case, said composition can be formulated, either as such or together pharmaceutically acceptable vehicles, in the form of tablets, capsules, pearls, plasters or in liquid form.

The pharmaceutically acceptable vehicles can be, for example, binding agents (such as pregelatinized corn starch, polyvinylpyrrolidone or hydroxypropylmethylcellulose); fillers (such as lactose, microcristalline cellulose or calcium hydrogen phosphate); lubrificants (such as magnesium stearate, talcum, or silica); disintegrants (such as potato starch or sodium starch glycolate); or inhibiting agents(such as sodium lauryl sulphate). The tablets can be coated by methods well known in the art.

The liquid preparations for oral administration can be, for example, in the form of solutions, syrups or suspensions or rather they can be freeze-dried products to be reconstituted, before use, with water or other suitable vehicles. These liquid preparations can be prepared through conventional methods with the pharmaceutically acceptable additives such as suspending agents (such as sorbitol syrup, cellulose derivatives or edible hydrogenated fats); emulsifying agents (such as lecithin or acacia); non-aqueous vehicles (such as almond oil, oil esters, ethylic alcohol or fractionated vegetable oils); and preservatives (such as methyl- or propyl-p-hydroxybenzoates or sorbic acid). The preparation may also suitably contain flavourings, colourings, and sweetening agents.

For buccal administration, the compositions can be in the form of conventionally formulated tablets or lozenges.

Preferably, the composition used is administered by oral or transdermal route in the form of tablets, capsules, pearls or in liquid form and plasters, respectively. Preferably, the liquid form is a syrup or freeze-dried product to be reconstituted before use with water or other suitable vehicles. More preferably, the inventive composition is formulated in the form of capsules or plaster.

According to the present invention, the composition dose (drug or food supplement) proposed for administration to a human (with a body weight of about 70 Kg) ranger from 10 mg to 500 mg of grape extract, preferably from 30 mg to 300 mg, more preferably from 40 mg to 200 mg per dose unit and from 40 mg to 800 mg of Monascus Fungus extract, preferably from 80 mg to 600 mg, more preferably from 120 mg to 400 mg per dose unit. The dose unit can be administered, for example, from 1 to 5 times a day, preferably 1 to 3 times a day, more preferably 1 to 2 times a day. The dose will also depend on the selected administration route. It should be considered that it may be required to carry out continuous variations of dosage according to the age and weight of the patient as well as the seriousness of the clinical condition to be treated. For example, higher doses may be provided in case of particularly serious diseases. Finally, the proper dose and administration rout will be at the discretion of the doctor or veterinary.

### CLINICAL TESTS

### Open study in humans (step I)

The inventive composition has been tested on 12 subjects with hypercholesterolemia, hypertrigliceridemia e hyperglycemia for 4 weeks. A one capsule per day dosage of 270 mg of Monascus, purpureus extract (0.8% monacolin), prepared according to the procedure described below, with 90 mg of commercial extracts of 95% grape seeds (titrated at 96.5% in proanthocyanidine) has been employed. The results are listed in Table 1 and Table 2.

**Table 1. Effect of the composition on the bloods lipid levels, glycemia and arterial pressure levels.**

| | | **Before treatment** | | | | | | **After treatment** | | | | | |
|---|---|---|---|---|---|---|---|---|---|---|---|---|---|
| **Name** | **Age** | **Chol Tot** | **LDL** | **HDL** | **TG** | **A.P.** | **Glyc** | **Chol tot** | **LDL** | **HDL** | **TG** | **A.P.** | **Glyc** |
| BZ | 71 | 258 | 191 | 44 | 115 | 140/70 | 176 | 195 | 140 | 42 | 66 | 130/75 | 143 |
| AD | 46 | 270 | 195 | 53 | 130 | 160/70 | 156 | 242 | 174 | 57 | 90 | 135/80 | 131 |
| LR | 76 | 293 | 230 | 48 | 186 | 135/90 | 114 | 253 | 182 | 57 | 136 | 125/90 | 120 |
| ST | 48 | 299 | 216 | 51 | 123 | 145/95 | 181 | 243 | 171 | 52 | 100 | 135/85 | 147 |
| RZ | 45 | 282 | 227 | 38 | 195 | 120/80 | 190 | 215 | 146 | 47 | 145 | 120/85 | 151 |
| FR | 64 | 259 | 146 | 93 | 97 | 115/80 | 156 | 220 | 104 | 92 | 90 | 120/80 | 125 |
| RM | 56 | 255 | 160 | 63 | 176 | 130/85 | 145 | 176 | 131 | 79 | 127 | 130/80 | 120 |
| MR | 65 | 238 | 147 | 39 | 257 | 150/85 | 162 | 207 | 139 | 41 | 185 | 130/80 | 139 |
| FS | 56 | 266 | 173 | 74 | 194 | 110/70 | 110 | 245 | 140 | 72 | 151 | 130/80 | 109 |
| GR | 68 | 319 | 241 | 50 | 537 | 125/90 | 109 | 233 | 161 | 56 | 361 | 125/85 | 111 |
| SI | 75 | 305 | 240 | 32 | 136 | 150/85 | 178 | 288 | 178 | 39 | 101 | 125/75 | 157 |
| VMT | 60 | 318 | | 65 | 200 | 130/80 | 165 | 260 | | 76 | 120 | 125/75 | 132 |

| | | | | | | | | | | | | | |
|---|---|---|---|---|---|---|---|---|---|---|---|---|---|
| Chol. Tot.= total cholesterol; LDL = LDL cholesterol; HDL = HDL cholesterol; TG = triglycerides; A.P. = arterial pressure; Glyc = glycemia. | | | | | | | | | | | | | |

After a 4-week treatment with the inventive composition, all patients have exhibited a substantial reduction in the total cholesterol, above all LDL cholesterol. The most marked effect has been obtained with triglycerides, mostly with those subjects having the highest values. The arterial pressure has also resulted to be reduced with the hypertensive subjects. In some cases, there has been obtained a decrease of glycaemia and in other cases the values have maintained steady. The inventive composition, hence, performs a controlling action on the glucose values in the blood.

**Table 2. Effect of the composition on the levels of the blood inflammation parameters.**

| | | **Before treatment** | | | | **After treatment** | | | |
|---|---|---|---|---|---|---|---|---|---|
| **Name** | **Age** | **CRP** | **Lp(a)** | **TNF- α** | **Co-Q** | **CRP** | **Lp(a)** | **TNF-α** | **Co-Q** |
| BZ | 71 | 2.30 | 279.5 | 298 | 1.27 | 1.59 | 221.9 | 206 | 1.22 |
| AD | 46 | 4.13 | 274.9 | 554 | 1.38 | 2.15 | 214.2 | 466 | 1.46 |
| LR | 76 | 1.70 | 234.9 | 305 | 0.98 | 1.30 | 199.4 | 261 | 1.08 |
| ST | 48 | 1.95 | 266.8 | 323 | 1.12 | 1.41 | 225.7 | 276 | 1.20 |
| RZ | 45 | 3.16 | 265.7 | 378 | 1.06 | 2.16 | 229.0 | 342 | 1.14 |
| FR | 64 | 3.98 | 304.2 | 385 | 0.99 | 2.03 | 246.8 | 321 | 1.10 |
| RM | 56 | 2.55 | 290.6 | 298 | 1.45 | 1.46 | 238.9 | 235 | 1.36 |
| MR | 65 | 2.78 | 311.3 | 288 | 1.23 | 1.81 | 243.1 | 270 | 1.28 |
| FS | 56 | 3.94 | 231.7 | 480 | 1.09 | 2.13 | 189.5 | 390 | 1.41 |
| GR | 68 | 3.72 | 276.0 | 412 | 1.11 | 2.12 | 221.0 | 341 | 1.31 |
| SI | 75 | 3.06 | 234.2 | 398 | 1.29 | 1.78 | 188.7 | 328 | 1.24 |
| VMT | 60 | 2.18 | | | 1.41 | 1.45 | | | 1.39 |

| | | | | | | | | | |
|---|---|---|---|---|---|---|---|---|---|
| CRP = C-reactive protein; Lp(a) = lipoprotein (a); TNF-α = Tumor Necrosis Factor-alfa Co-Q = coenzyme Q | | | | | | | | | |

The inventive composition determines after a 4-week treatment a substantial decrease in all arterial inflammation parameters and at the same time does not cause the depletion of endogenous anti-oxidants i.e. the coenzyme Q.

### How to grown the Monascus Fungus and prepare the extract.

The commercial Monascus Purpureos, obtained from freeze-dried powder, in a glass ampoule in vacuum, has been first grown in potato dextrose agar (PDA, Difco Laboratories, USA) to induce the formation of spores. After culture (30°C for 7 days) the colonies (∼100 mm²) have been transferred in 100 ml of potato dextrose bouillon (PDB, Difco Laboratories, USA) and incubated (30°C for 4 days) under stirring conditions (2,5 Hz). After incubation, 1,25 ml (5% V/V) of the above bouillon has been incubated with 25 ml of rice-glycerol complex medium, with different concentrations of rice powder, peptone, glycerol and glucose in a 250 ml flask (Minori). Culture medium ingredients in g/L: KNO₃ 8, MgSO₄•7H₂O 4. The culture (pH 5) has been incubated (25°C, 10 gg) under stirring (2,5 Hz). Extraction: at the completion of growth, the whole bouillon has been filtered and the remaining solid extracted with 95% ethanol (50 ml per 250 ml flask) for 1 day. Subsequently, the extract has been filtered through a 0,45 pm membrane and evaporated in vacuum. The amount of lovastatin has been determined according to the procedure below.

### Determination of Lovastatin

Standard Test: Code according to the Chinese Ministry of Health: WS-055-97.

### Method: HPLC

Conditions: chromatography column: ODSC18, 4.6*150mm; mobile phase: acetonitrile/0,1%, phosphoric acid 65/35; wavelength for UV testing: 238 nm; injection rate: 1 ml/min; injection amount 10 µl; solvent: 95% ethanol.

## Claims

1. Use of a composition comprising a Monascus fungus extract and grape extract for preparing a medicament for the treatment and prevention of the cardiovascular system inflammation.

2. Use of a composition comprising a Monascus fungus extract and grape extract for preparing a medicament for the treatment and prevention of hypertension.

3. Use a composition comprising a Monascus fungus extract and grape extract for the treatment and prevention of the following diseases: hyperlipidemia, cardiac diseases, hypertension, atherosclerosis and metabolic disequilibrium.

4. Use according to any claim 1 to 3 wherein said composition is formulated either as such or together pharmaceutically acceptable excipients.

5. Use according to claims 1 to 4, wherein said grape extract is a grape seed extract.

6. Use according to claim 5, wherein said grape extract comprises 1% to 99% by weight of polyphenols.

7. Use according to any claims 5 or 6, wherein said grape extract comprises 50% to 99% by weight, preferably 70% to 99% by weight of polyphenols.

8. Use according to claims 5 to 7, wherein said polyphenols comprise 1% to 95% by weight of proanthocyanidins and 5% to 99% by weight of anthocyanosides.

9. Use according to claims 5 to 8, wherein said polyphenols comprise 70% to 95% by weight, preferably 80% to 95% by weight of proanthocyanidins and 5% to 30% by weight of anthocyanosides, preferably 5% to 20% by weight.

10. Use according to any claim 5 to 9, wherein said grape extract is extracted from any plant belonging to the family of Vitaceae, preferably it is Vitis vinifera extract.

11. Use according to any claim 5 to 10, wherein said grape extract is extracted from grape seeds.of Vitis vinifera.

12. Use according to any claim 5 to 11, wherein said Monascus fungus extract comprises monacolins and a further moiety comprising, inter alia: polyunsaturated fatty acids, phytosterols (es: β-sitosterol , capesterol, stigmasterol), isoflavons and the glucosides thereof, saponins and red pigments.

13. Use according to any claim 5 to 12, wherein said Monascus fungus extract comprises monacolin in amounts from 0,01% to 10% by weight, preferably 0,2% to 5% by weight.

14. Use according to any claim 5 to 13, wherein said Monascus fungus extract comprises monacolin in amounts from 0,3% to 2% by weight.

15. Use according to any claim 5 to 14, wherein said Monascus fungus extract is extracted from any Monascus fungus species.

16. Use according to any claim 5 to 15, wherein said Monascus fungus extract is extracted from one or more species selected from the following group: Monascus albidus Sato; Monascus pilosus Sato; Monascus pubigereus Sato; Monascus ruber van Tieghen; Monascus paxii Liengelsheim; Monascus fulginosus Sato; Monascus purpueus Went; preferably Monascus purpureus Went, Monascus ruber van Tieghen, Monascus fulginosus Sato, Monascus albidus Sato.

17. Use according to any claim 5 to 18, wherein said Monascus fungus extract is extracted from the following group: Monascus purpureus Went, Monascus ruber van Tieghen; preferably Monascus purpureus Went.

18. Use according to any claim 5 to 17, wherein the composition comprises Monascus fungus extract in amounts ranging from 7% to 99% by weight and grape extract ranging from 1% to 93% by weight.

19. Use according to any claim 5 to 18, wherein the composition comprises Monascus fungus extract in amounts ranging from 21% to 95% by weight, preferably from 37% to 91% by weight and grape extract from 5% to 79% by weight, preferably from 9% to 63% by weight.

20. Use according to any claim 1 to 19, wherein said composition is formulated either as such or together pharmaceutically acceptable excipients.

21. Use according to any claim 1 to 20, wherein said composition is administered by oral, transdermal or buccal route.

22. Use according to any claim 1 to 21, wherein said composition is administered by oral or transdermal route.

23. Use according to any claim 1 or 22, wherein said composition is administered in the form of tablets, capsules, pearls, plasters or in liquid form, preferably in the form of capsules or plasters.

24. Use according to any claim 1 to 23, wherein said composition comprises 10 mg to 500 mg of grape extract and 40 mg to 800 mg of Monascus fungus extract per dose unit.

25. Use according to any claim 1 to 24, wherein said composition comprises 30 mg to 300 mg, preferably 40 mg to 200 mg of grape extract and 80 mg to 600 mg, preferably 120 mg to 400 mg of Monascus fungus extract per dose unit.

26. Use according to claim 24 or 25 wherein said dose unit is administered 1 to 5 times a day, preferably 1 to 3 times a day.

27. Use according to claim 26 wherein said dose unit is administered 1 to 2 times a day.

## Patentansprüche

1. Verwendung einer Zusammensetzung, die einen Monascus-Pilz-Extrakt und einen Traubenextrakt umfasst, zur Herstellung eines Medikaments zur Behandlung und Prävention der Ansammlung von hohen Zuckerspiegeln im Blut.

2. Verwendung einer Zusammensetzung, die einen Monascus-Pilz-Extrakt und einen Traubenextrakt umfasst, zur Herstellung eines Medikaments zur Behandlung und Prävention einer Entzündung des kardiovaskulären Systems.

3. Verwendung einer Zusammensetzung, die einen Monascus-Pilz-Extrakt und einen Traubenextrakt umfasst, zur Herstellung eines Medikaments zur Behandlung und Prävention von Hypertonie.

4. Verwendung nach einem der Ansprüche 1 bis 3 zur Behandlung und Prävention der folgenden Erkrankungen: Hyperlipidämie, Herzerkrankungen, Hypertonie, Atherosklerose und metabolisches Ungleichgewicht.

5. Verwendung nach einem der Ansprüche 1 bis 4, wobei der Traubenextrakt ein Traubenkernextrakt ist.

6. Verwendung nach Anspruch 5, wobei der Traubenextrakt 1 bis 99 Gew.-% an Polyphenolen umfasst.

7. Verwendung nach Anspruch 5 oder 6, wobei der Traubenextrakt 50 bis 99 Gew.-%, vorzugsweise 70 bis 99 Gew.-% an Polyphenolen umfasst.

8. Verwendung nach den Ansprüchen 5 bis 7, wobei die Polyphenole 1 bis 95 Gew.-% an Proanthocyanidinen und 5 bis 99 Gew.-% an Anthocyanosiden umfassen.

9. Verwendung nach den Ansprüchen 5 bis 8, wobei die Polyphenole 70 bis 95 Gew.-%, vorzugsweise 80 bis 95 Gew.-% an Proanthocyanidinen und 5 bis 30 Gew.-%, vorzugsweise 5 bis 20 Gew.-% an Anthocyanosiden umfassen.

10. Verwendung nach den Ansprüchen 5 bis 9, wobei der Traubenextrakt aus einer Pflanze extrahiert ist, die zur Familie der Vitaceae gehört, vorzugsweise ein *Vitis vinifera-*Extrakt ist.

11. Verwendung nach den Ansprüchen 5 bis 10 , wobei der Traubenextrakt aus Traubenkernen von *Vitis vinifera* extrahiert ist.

12. Verwendung nach den Ansprüchen 5 bis 11, wobei der Monascus-Pilz-Extrakt Monacoline und eine weitere Einheit umfasst, wobei die weitere Einheit unter anderem mehrfach ungesättigte Fettsäuren, Phytosterine (insbesondere β-Sitosterin, Campesterin, Stigmasterin), Isoflavone und die Glucoside derselben, Saponine und rote Pigmente umfasst.

13. Verwendung nach den Ansprüchen 5 bis 12, wobei der Monascus-Pilz-Extrakt Monacolin in Mengen von 0,01 bis 10 Gew.-%, vorzugsweise 0,2 bis 5 Gew.-% umfasst.

14. Verwendung nach den Ansprüchen 5 bis 13, wobei der Monascus-Pilz-Extrakt Monacolin in Mengen von 0,3 bis 2 Gew.-% umfasst.

15. Verwendung nach den Ansprüchen 5 bis 14, wobei der Monascus-Pilz-Extrakt aus einer beliebigen Monascus-Pilzart extrahiert ist.

16. Verwendung nach den Ansprüchen 5 bis 15, wobei der Monascus-Pilz-Extrakt aus einer oder mehreren Arten extrahiert ist, die aus der folgenden Gruppe ausgewählt sind: *Monascus albidus* Sato, *Monascus pilosus* Sato, *Monascus pubigerus* Sato, *Monascus ruber* van Tieghen, *Monascus paxii* Liengelsheim, *Monascus fuliginosus* Sato, *Monascus purpureus* Went; vorzugsweise *Monascus purpureus* Went, *Monascus ruber* van Tieghen, *Monascus fuliginosus* Sato, *Monascus albidus* Sato.

17. Verwendung nach den Ansprüchen 5 bis 16, wobei der Monascus-Pilz-Extrakt aus der folgenden Gruppe ausgewählt ist: *Monascus purpureus* Went, *Monascus ruber* van Tieghen; vorzugsweise *Monascus purpureus* Went.

18. Verwendung nach den Ansprüchen 5 bis 17, wobei die Zusammensetzung einen Monascus-Pilz-Extrakt in Mengen im Bereich von 7 bis 99 Gew.-% und einen Traubenextrakt im Bereich von 1 bis 93 Gew.-% umfasst.

19. Verwendung nach den Ansprüchen 5 bis 18, wobei die Zusammensetzung einen Monascus-Pilz-Extrakt in Mengen im Bereich von 21 bis 95 Gew.-%, vorzugsweise 37 bis 91 Gew.-% und einen Traubenextrakt im Bereich von 5 bis 79 Gew.-%, vorzugsweise 9 bis 63 Gew.-% umfasst.

20. Verwendung nach einem der Ansprüche 1 bis 19, wobei die Zusammensetzung entweder als solche oder zusammen mit pharmazeutisch akzeptablen Streckmitteln formuliert ist.

21. Verwendung nach einem der Ansprüche 1 bis 20, wobei die Zusammensetzung auf oralem, transdermalem oder bukkalem Weg verabreicht wird.

22. Verwendung nach einem der Ansprüche 1 bis 21, wobei die Zusammensetzung auf oralem oder transdermalem Weg verabreicht wird.

23. Verwendung nach einem der Ansprüche 1 oder 22, wobei die Zusammensetzung in der Form von Tabletten, Kapseln, Perlen, Pflastern oder in flüssiger Form, vorzugsweise in der Form von Kapseln oder Pflastern verabreicht wird.

24. Verwendung nach einem der Ansprüche 1 bis 23, wobei die Zusammensetzung 10 mg bis 500 mg Traubenextrakt und 40 mg bis 800 mg Monascus-Pilz-Extrakt pro Dosiseinheit umfasst.

25. Verwendung nach einem der Ansprüche 1 bis 24, wobei die Zusammensetzung 30 mg bis 300 mg, vorzugsweise 40 mg bis 200 mg Traubenextrakt und 80 mg bis 600 mg, vorzugsweise 120 mg bis 400 mg Monascus-Pilz-Extrakt pro Dosiseinheit umfasst.

26. Verwendung nach Anspruch 24 oder 25, wobei die Dosiseinheit ein- bis fünfmal pro Tag, vorzugsweise ein- bis dreimal pro Tag verabreicht wird.

27. Verwendung nach Anspruch 26, wobei die Dosiseinheit einbis zweimal pro Tag verabreicht wird.

## Revendications

1. Utilisation d'une composition comprenant un extrait de champignon Monascus et un extrait de raisin pour préparer un médicament pour le traitement et la prévention de l'accumulation de hauts niveaux de sucres dans le sang.

2. Utilisation d'une composition comprenant un extrait de champignon Monascus et un extrait de raisin pour préparer un médicament pour le traitement et la prévention de l'inflammation du système cardiovasculaire.

3. Utilisation d'une composition comprenant un extrait de champignon Monascus et un extrait de raisin pour préparer un médicament pour le traitement et la prévention de l'hypertension.

4. Utilisation selon l'une quelconque des revendications 1 à 3 pour le traitement et la prévention des maladies suivantes : hyperlipidémie, maladies cardiaques, hypertension, athérosclérose et déséquilibre métabolique.

5. Utilisation selon l'une quelconque des revendications 1 à 4 où ledit extrait de raisin est un extrait de pépins de raisin.

6. Utilisation selon la revendication 5 où ledit extrait de raisin comprend 1 % à 99 % en poids de polyphénols.

7. Utilisation selon la revendication 6 ou 5 où ledit extrait de raisin comprend 50 % à 99 % en poids, de préférence 70 % à 99 % en poids de polyphénols.

8. Utilisation selon les revendications 5 à 7 où lesdits polyphénols comprennent 1 % à 95 % en poids de proanthocyanidines et 5 % à 99 % en poids d'anthocyanosides.

9. Utilisation selon les revendications 5 à 8 où lesdits polyphénols comprennent 70 % à 95 % en poids, de préférence 80 % à 95 % en poids de proanthocyanidines et 5 % à 30 % en poids d'anthocyanosides, de préférence 5 % à 20 % en poids.

10. Utilisation selon les revendications 5 à 9 où ledit extrait de raisin est extrait de toute plante appartenant à la famille des Vitaceae, de préférence c'est un extrait de Vitis vinifera.

11. Utilisation selon les revendications 5 à 10 où ledit extrait de raisin est extrait de pépins de raisin de Vitis vinifera.

12. Utilisation selon les revendications 5 à 11 où ledit extrait de champignon Monascus comprend des monacolines et une autre entité comprenant, entre autres : des acides gras polyinsaturés, des phytostérols (par exemple : β-sitostérol, capéstérol, stigmastérol), des isoflavones et leurs glucosides, des saponines et des pigments rouges.

13. Utilisation selon les revendications 5 à 12 où ledit extrait de champignon Monascus comprend de la monacoline en des quantités de 0,01 % à 10 % en poids, de préférence de 0,2 % à 5 % en poids.

14. Utilisation selon les revendications 5 à 13 où ledit extrait de champignon Monascus comprend de la monacoline en des quantités de 0,3 % à 2 % en poids.

15. Utilisation selon les revendications 5 à 14 où ledit extrait de champignon Monascus est extrait de toute espèce de champignon Monascus.

16. Utilisation selon les revendications 5 à 15 où ledit extrait de champignon Monascus est extrait d'une ou plusieurs espèces choisies dans le groupe suivant : Monascus albidus Sato ; Monascus pilosus Sato ; Monascus pubigereus Sato ; Monascus ruber van Tieghen ; Monascus paxii Liengelsheim ; Monascus fulginosus Sato ; Monascus purpureus Went ; de préférence Monascus purpureus Went, Monascus ruber van Tieghen, Monascus fulginosus Sato, Monascus albidus Sato.

17. Utilisation selon les revendications 5 à 16 où ledit extrait de champignon Monascus est extrait du groupe suivant : Monascus purpureus Went, Monascus ruber van Tieghen, de préférence Monascus purpureus Went.

18. Utilisation selon les revendications 5 à 17 où la composition comprend un extrait de champignon Monascus en des quantités allant de 7 % à 99 % en poids et un extrait de raisin allant de 1 % à 93 % en poids.

19. Utilisation selon les revendications 5 à 18 où la composition comprend un extrait de champignon Monascus en des quantités allant de 21 % à 95 % en poids, de préférence de 37 % à 91 % en poids et un extrait de raisin de 5 % à 79 % en poids, de préférence de 9 % à 63 % en poids.

20. Utilisation selon l'une quelconque des revendications 1 à 19 où ladite composition est formulée en l'état ou avec des excipients pharmaceutiquement acceptables.

21. Utilisation selon l'une quelconque des revendications 1 à 20 où ladite composition est administrée par voie orale, transdermique ou buccale.

22. Utilisation selon l'une quelconque des revendications 1 à 21 où ladite composition est administrée par voie orale ou transdermique.

23. Utilisation selon l'une quelconque des revendications 1 à 22 où ladite composition est administrée sous forme de comprimés, de capsules, de perles, d'emplâtres ou sous forme liquide, de préférence sous forme de capsules ou d'emplâtres.

24. Utilisation selon l'une quelconque des revendications 1 à 23 où ladite composition comprend 10 mg à 500 mg d'extrait de raisin et 40 mg à 800 mg d'extrait de champignon Monascus par dose unitaire.

25. Utilisation selon l'une quelconque des revendications 1 à 24 où ladite composition comprend 30 mg à 300 mg, de préférence 40 mg à 200 mg d'extrait de raisin et 80 mg à 600 mg, de préférence 120 mg à 400 mg d'extrait de champignon Monascus par dose unitaire.

26. Utilisation selon la revendication 24 ou 25 où ladite dose unitaire est administrée 1 à 5 fois par jour, de préférence 1 à 3 fois par jour.

27. Utilisation selon la revendication 26 où ladite dose unitaire est administrée 1 à 2 fois par jour.
